# FASCICULE DE BREVET EUROPEEN

(11) **EP 2 287 274 B1**
(45) Date de publication et mention de la délivrance du brevet: **13.03.2013**
(21) Numéro de dépôt: 10290402.6
(22) Date de dépôt: 16.07.2010
(51) Int. Cl.: C10G 45/36, B01J 23/835, B01J 23/89, B01J 37/02, C07C 5/05

(54) **Procédé de préparation d'un catalyseur supporte Ni/Sn pour l'hydrogénation sélective d'hydrocarbures polyinsaturés**
Verfahren zur Herstellung eines Ni/Sn-unterstützten Katalysators für das selektive Hydrierverfahren von mehrfach ungesättigten Kohlenwasserstoffen
Method for preparing a Ni/Sn support catalyst for selective hydrogenation of polyunsaturated hydrocarbons

(30) Priorité: 17.08.2009 FR 0903988
(43) Date de publication de la demande: 23.02.2011
(73) Titulaire: IFP Energies nouvelles, 92852 Rueil-Malmaison Cedex (FR)
(72) Inventeur: Fischer, Lars, 38200 Veinne (FR); Dubreuil, Anne-Claire, 69003 Lyon (FR); Thomazeau, Cecile, 69008 Lyon (FR); Deghedi, Layane, 69007 Lyon (FR); Candy, Jean-Pierre, 69300 Caluire et Cuire (FR); Basset, Jean-Marie, 69300 Caluire et Cuire (FR); Le Peltier, Fabienne, 92500 Rueil Malmaison (FR)

(56) Documents cités:
- FR-A1- 2 539 647
- FR-A1- 2 698 351
- FR-A1- 2 792 645
- US-B1- 6 566 573

## Description

### Domaine technique

La présente invention concerne l'hydrogénation sélective de composés polyinsaturés présents dans une charge d'hydrocarbures comme par exemple dans les essences de vapocraquage. Ces essences contiennent en effet des composés générateurs de gommes, notamment des dioléfines et alkénylaromatiques, en mélange notamment avec des composés mono-oléfiniques et des composés aromatiques. Pour pouvoir valoriser les essences de vapocraquage, celles-ci ont besoin d'être débarrassées de leur teneur en dioléfines respectivement en alkénylaromatiques, les dioléfines étant hydrogénées sélectivement en mono-oléfines et les alkénylaromatiques étant hydrogénés sélectivement en aromatiques. De manière plus précise, l'invention se rapporte à un procédé de préparation d'un catalyseur supporté contenant de l'étain et du nickel pour l'hydrogénation sélective des composés polyinsaturés présents dans les coupes hydrocarbures.

### Art antérieur

Les traitements par hydrogénation sélective sont généralement effectués sur des catalyseurs métalliques déposés sur un support amorphe ou cristallin. Les métaux utilisés sont les métaux du groupe VIII et parmi ceux-ci, on peut noter le nickel qui est d'un usage courant.

Cependant les catalyseurs au nickel ne sont pas suffisamment sélectifs car ils ont une tendance marquée à hydrogéner une part importante des mono-oléfines contenues dans la charge, même lorsque les hydrogénations sont effectuées à basse pression, de l'ordre de 30 à 50 bar et à basse température, entre 50°C et 180°C (degrés Celsius).

Il est connu que l'amélioration de la sélectivité de ces catalyseurs peut être obtenue en injectant des composés sulfurés avant la mise en contact du catalyseur avec la charge réactive de manière à obtenir un catalyseur passivé au soufre. Ces composés peuvent être choisis parmi les composés suivants: thiophène, thiophane, alkylmonosulfures tels que diméthylsulfure, diéthylsulfure, dipropylsulfure, propylméthyl-sulfure. Cette sulfuration est cependant délicate à réaliser car il est nécessaire que le composé sulfuré soit très également réparti sur l'ensemble du lit catalytique pour que l'on puisse effectivement obtenir un effet marqué sur la sélectivité. De plus cette procédure est coûteuse et longue, ce qui se traduit en perte de production.

Le manque de sélectivité intrinsèque du catalyseur au nickel ne se manifeste pas seulement vis-à-vis des mono-oléfines, il se manifeste également vis-à-vis des aromatiques. Si la surface des particules de Ni était passivée en surface par des composés organiques soufrés de manière très contrôlée, *a priori* sur des sites de Ni bien distincts, et à raison d'un atome de S par environ 4 ou 5 atomes de Ni de surface [T.E. Fischer, S.R. Kelemen, J. Catal., 53, 24, (1978), ou GB1565754], l'hydrogénation des noyaux aromatiques serait supprimée et l'hydrogénation des composés mono-oléfiniques serait fortement ralentie. La maîtrise à l'échelle industrielle du dépôt de soufre aussi contrôlé, ainsi que la stabilité de ce système dans des conditions réactionnelles utilisées pour les hydrogénations sélectives (25 - 30 bar, 50 - 180°C), est toutefois difficile à atteindre. C'est ainsi qu'au démarrage d'un catalyseur au nickel, même passivé par un composé sulfuré du type de ceux indiqués ci-dessus, il est nécessaire d'utiliser une charge de démarrage non réactive, ne contenant ni dioléfine, ni mono-oléfine et très peu d'aromatiques. En effet l'hydrogénation des dioléfines ou éventuellement des mono-oléfines sur les catalyseurs neufs qui sont très actifs, provoque un dégagement de chaleur suffisant pour élever la température du catalyseur à des niveaux bien supérieurs à 200°C, ce qui peut provoquer l'hydrogénation des aromatiques. Cette dernière réaction est encore plus exothermique et la température peut dépasser 600°C, ce qui a pour résultat de provoquer le craquage des hydrocarbures, une réaction elle-aussi très exothermique. C'est ainsi que les températures atteintes lors de ces emballements peuvent dépasser les températures de calcul des réacteurs en acier, ce qui oblige à remplacer non seulement la charge de catalyseur mais aussi le réacteur lui-même.

Une autre méthode d'amélioration de la sélectivité catalytique consiste à pré-passiver le catalyseur au Ni à l'état oxydé et en l'absence d'hydrogène, par imprégnation avec un composé organique du soufre, par exemple le 2,2 dithio bis éthanol (D.E.O.D.S), tel que décrit dans le brevet EP0466567. Le polysulfure se décompose sous hydrogène dans le réacteur d'hydrogénation, simultanément à la réduction du nickel, et l'introduction d'une charge réactive peut se faire par la suite sans danger d'emballement de la réaction. Toutefois, étant donné que la réduction et la passivation au soufre ont lieu en même temps, il est encore plus difficile dans ces conditions d'obtenir une passivation homogène de la surface du Ni avec la stoechiométrie souhaitée et uniquement sur les sites de Ni souhaités. Pour éviter la présence de particules de Ni réduit non passivé, le polysulfure est introduit en excès et une sur-passivation avec formation partielle de la phase Ni₃S₂ ne peut, en général, pas être évitée, ce qui diminue de façon significative l'activité catalytique même envers les dioléfines (B.W. Hoffer, R.L.C. Bonne, A.D. van Langeveld, C. Griffiths, C.M. Lok, J.A. Moulijn, Fuel 83 (2004) 1-8).

Il est également connu que les catalyseurs bimétalliques peuvent apporter des gains de sélectivité et de stabilité. Pour des catalyseurs d'hydrogénation sélective, il est connu que des associations du palladium avec un deuxième métal rendent les catalyseurs plus sélectifs mais moins actifs. De telles associations ont seulement été proposées pour les réactions d'hydrogénations sélectives des coupes contenant des hydrocarbures ayant entre deux et quatre atomes de carbone, pour lesquelles souvent un premier réacteur travaille avec un catalyseur monométallique au palladium pour effectuer la majeur partie de la conversion, et un deuxième réacteur contenant un catalyseur bimétallique complète la conversion de manière plus sélective. Par exemple, le brevet US5356851 enseigne qu'il est avantageux d'associer un métal du groupe VIII (préférentiellement le palladium) à un élément tel que l'indium ou le gallium pour des applications en hydrogénation sélective de composés polyinsaturés. De même des associations Pd-Cu (US5464802), Pd-Ag (US4547600), Pd-Sn et Pd-Pb (JP59227829) ou encore une combinaison de palladium et d'un métal alcalin (EP0722776) ont été identifiées pour leur performances en hydrogénation. L'ensemble de ces brevets vise une augmentation du rendement en mono-oléfines.

Des catalyseurs bimétalliques à base de nickel sont aussi décrits dans l'art antérieur. Le brevet GB1565754 décrit l'introduction de 10ppm d'un élément du groupe du platine, tel que le ruthénium, le rhodium, le palladium, l'osmium, l'iridium et le platine, et de façon préférée de palladium, dans un catalyseur d'hydrogénation sélective de coupes C3, C4 ou essence contenant 10% de Ni supporté sur sépiolite. Cette introduction a pour effet d'augmenter la réductibilité du Ni après régénération, mais l'introduction ultérieure de composés sulfures pour la passivation est toujours nécessaire. Le brevet US5997835 décrit l'introduction d'or dans un catalyseur au nickel supporté par une méthode en phase aqueuse pour le vaporeformage du méthane, l'effet de l'introduction de or étant de ralentir la désactivation du catalyseur.

Le brevet FR2792645 décrit la préparation en phase aqueuse à pH inférieur à 10 d'un catalyseur bimétallique associant un métal du groupe VIII (préférentiellement le platine, le palladium et le nickel) et un métal préférentiellement choisi parmi le germanium, l'étain, l'argent et l'or. Le brevet FR2539647 décrit l'introduction de germanium, d'étain, ou de plomb, sous forme d'une solution d'un alkyle de ce métal, et d'un métal du groupe VIII (préférentiellement le platine, le palladium, le nickel et le cobalt). Ces catalyseurs présentent un intérêt pour des applications en hydrogénation sélective de composés polyinsaturés, dans un but d'augmenter le rendement en mono-oléfines, ou pour des applications d'hydrodésulfuration.

Le brevet FR2698351 enseigne qu'il est avantageux d'associer un métal du groupe VIII choisi parmi l'iridium, l'osmium, le nickel, le palladium, le platine et le rhodium (le rhodium étant le métal préféré) et un métal additionnel choisi dans le groupe IVa constitué par l'étain, le germanium et le plomb pour l'hydrogénation sélective du 4-vinyl-cyclohexène en vinyl-cyclohexane. La méthode de préparation préférée consiste à préparer un catalyseur monométallique à base du métal du groupe VIII, à le réduire, puis à l'imprégner soit en solution aqueuse, soit en solution organique, soit en phase gaz, par un composé du métal du groupe IVa. Le produit obtenu peut éventuellement être réduit avant utilisation. Les pourcentages des métaux mis en oeuvre sont compris préférentiellement entre 1 % poids et 3 % poids pour les métaux du groupe VIII et entre 0,01 % poids et 15 % poids pour le métal additionnel, ce qui correspond à un rapport molaire élément du groupe VIII sur élément du groupe IVa compris entre 0,3 et 3.

La présente invention a pour objectif de fournir un catalyseur contenant une phase métallique à base de nickel et d'étain, préparé par un nouveau procédé de préparation, pour l'hydrogénation sélective de composés polyinsaturés présents dans une coupe hydrocarbonée. Plus précisément, elle se propose de fournir un catalyseur alternatif aux catalyseurs à base de nickel passivés au soufre connus de l'art antérieur.

### Résumé et intérêt de l'invention

La présente invention a pour objet un procédé de préparation d'un catalyseur comprenant au moins un support poreux et au moins une phase métallique contenant du nickel et de l'étain dans une proportion telle que le rapport molaire Sn/Ni est compris entre 0,01 et 0,2, ledit procédé comprenant au moins successivement les étapes suivantes :
a) le dépôt de nickel sur au moins ledit support pour obtenir un catalyseur supporté monométallique à base de nickel,
b) la réduction dudit catalyseur monométallique en présence d'au moins un gaz réducteur,
c) le dépôt, en phase gazeuse et en présence d'au moins un gaz réducteur, d'au moins un composé organométallique de l'étain sur ledit catalyseur monométallique réduit et,
d) l'activation du solide issu de ladite étape c) en présence d'au moins un gaz réducteur pour obtenir un catalyseur supporté ayant une phase métallique à base de nickel et d'étain dans lequel l'étain représente de 0,02 à 15 % poids de la masse dudit catalyseur et le nickel de 1 à 50% poids de la masse du catalyseur.

La présente invention a également pour objet un procédé d'hydrogénation sélective d'une charge d'hydrocarbures polyinsaturés, ledit procédé comprenant le passage de ladite charge dans au moins une unité réactionnelle pourvue d'au moins un catalyseur supporté comprenant au moins une phase métallique contenant de l'étain et du nickel préparé selon le procédé de préparation de l'invention.

Le catalyseur, préparé selon le procédé de l'invention et mis en oeuvre dans un procédé d'hydrogénation sélective d'hydrocarbures polyinsaturés, conduit à des performances catalytiques améliorées en terme de sélectivité envers les composés mono-insaturés. De plus, le catalyseur, préparé selon le procédé de l'invention et mis en oeuvre dans un procédé d'hydrogénation sélective d'hydrocarbures polyinsaturés, limite sensiblement voire supprime l'hydrogénation des noyaux aromatiques présents dans des composés polyinsaturés tels que les composés aromatiques, styréniques ou indéniques, ce qui permet d'éviter tout emballement des réactions et de valoriser les composés présentant un noyau aromatique, issus dudit procédé d'hydrogénation sélective selon l'invention, dans diverses applications.

### Description de l'invention

La présente invention a pour objet un procédé de préparation d'un catalyseur comprenant au moins un support poreux et au moins une phase métallique contenant du nickel et de l'étain dans une proportion telle que le rapport molaire Sn/Ni est compris entre 0,01 et 0,2, ledit procédé comprenant au moins successivement les étapes suivantes :
a) le dépôt de nickel sur au moins ledit support pour obtenir un catalyseur supporté monométallique à base de nickel,
b) la réduction dudit catalyseur monométallique en présence d'au moins un gaz réducteur,
c) le dépôt, en phase gazeuse et en présence d'au moins un gaz réducteur, d'au moins un composé organométallique de l'étain sur ledit catalyseur monométallique réduit et,
d) l'activation du solide issu de ladite étape c) en présence d'au moins un gaz réducteur pour obtenir un catalyseur supporté ayant une phase métallique à base de nickel et d'étain dans lequel l'étain représente de 0,02 à 15 % poids de la masse dudit catalyseur et le nickel de 1 à 50% poids de la masse dudit catalyseur.

Conformément au procédé de préparation de l'invention, il est essentiel que lesdites étapes a), b), c) et d) soient réalisées successivement l'une après l'autre. Toutefois, des étapes intermédiaires peuvent être réalisées entre chacune desdites étapes a), b), c) et d) dès lors que l'ordre de réalisation des étapes a) à d) est maintenu.

Conformément à l'invention, le catalyseur préparé par le procédé de l'invention contient du nickel et de l'étain déposés sur un support poreux. La teneur en nickel est généralement comprise entre 1 % poids et 50 % poids, de manière préférée entre 5 % poids et 40 % poids et de manière encore plus préférée entre 8 % poids et 30 % poids de la masse du catalyseur. La teneur en étain est généralement comprise entre 0,02 % poids et 15 % poids de la masse du catalyseur. Le rapport molaire Sn/Ni est généralement compris entre 0,01 et 0,2, de façon préférée entre 0,025 et 0,055, et de façon encore plus préférée entre 0,03 et 0,05.

Le support poreux présent dans le catalyseur préparé selon le procédé de l'invention comprend généralement au moins un oxyde réfractaire qui est généralement sélectionné parmi les oxydes de métaux des groupes 2, 3, 4, 13 et 14 de la nouvelle classification périodique des éléments tel que par exemple les oxydes de magnésium, d'aluminium, de silicium, de titane, de zirconium, de thorium pris seuls ou en mélange entre eux ou en mélange avec d'autres oxydes de métaux de la classification périodique. On peut aussi utiliser le charbon. Le support préféré est choisi parmi les alumines, les silices ou les silices-alumines, et de façon encore plus préférée il s'agit d'une alumine ou d'une silice. Le volume poreux du support est généralement compris entre 0,1 cm³/g et 1,5 cm³/g, de préférence compris entre 0,5 cm³/g et 1 cm³/g. La surface spécifique du support est généralement comprise entre 10 m²/g et 250 m²/g, de préférence entre 30 m²/g et 200 m²/g et de façon encore plus préférée entre 40 m²/g et 180 m²/g. Ledit support poreux se présente avantageusement sous forme de billes, d'extrudés, de pastilles, ou d'agglomérats irréguliers et non sphériques dont la forme spécifique peut résulter d'une étape de concassage. De manière très avantageuse, ledit support se présente sous forme de billes ou d'extrudés.

Conformément à l'étape a) du procédé de préparation selon l'invention, le nickel est déposé sur le support poreux. Le dépôt du nickel sur ledit support peut être effectué par toute méthode connue de l'homme du métier. Par exemple, le dépôt est réalisé par imprégnation consistant en la mise en contact dudit support poreux avec au moins une solution aqueuse ou organique d'au moins un composé du nickel ou avec une suspension d'au moins un composé organique ou inorganique de nickel, ou bien le dépôt est réalisé par des méthodes de déposition - précipitation bien connues de l'Homme du métier [J.W. Geus, Préparation of Catalysts III, dans G. Poncelet, P. Grange, P.A. Jacobs (Eds.), Elsevier, Amsterdam 1983, 1]. Le dépôt du nickel sur le support est suivi éventuellement par un ou plusieurs lavages et/ou éventuellement par une évaporation du solvant. De manière avantageuse, le dépôt du nickel sur le support poreux est suivi d'un ou plusieurs traitements thermiques ou chimiques conduisant à un catalyseur supporté monométallique à base de nickel à l'état majoritairement oxyde ou majoritairement métallique. Ladite étape a) conduit à la préparation d'un catalyseur supporté monométallique à base de nickel.

Conformément à l'étape b) du procédé selon l'invention, on procède à au moins une étape de réduction dudit catalyseur monométallique, issu de ladite étape a), en présence d'un gaz réducteur, de préférence l'hydrogène. Ladite étape b) est effectuée à une température comprise entre 100°C et 600°C, de manière préférée entre 200°C et 500°C pendant une durée comprise entre 1 heure et 40 heures, de manière préférée entre 5 heures et 30 heures. La montée jusqu'à cette température de réduction est généralement lente, par exemple elle est fixée entre 0,1°C/minute et 5°C/minute. Ces conditions permettent d'obtenir un catalyseur supporté monométallique très majoritairement réduit.

Conformément à l'étape c) du procédé selon l'invention, on procède à au moins une étape de dépôt, en phase gazeuse et en présence d'au moins un gaz réducteur, d'au moins un composé organométallique de l'étain sur ledit catalyseur monométallique réduit. Le gaz réducteur est préférentiellement l'hydrogène. Ladite étape c) est effectuée à une température comprise entre 10°C et 100°C, de préférence comprise entre 20°C et 50°C, et pendant une durée comprise entre 50 minutes et 5 heures. L'étape c) est réalisée en l'absence de solvant.

Le composé organométallique de l'étain employé pour la mise en oeuvre de ladite étape c) est généralement sélectionné dans le groupe constitué par les alkyl-étain et les aryl-étain. Ainsi ledit composé organométallique de l'étain répond de préférence à la formule Sn(R)ₙH₄₋ₙ où R est un radical alkyle ou un radical aryle ayant de 1 à 12 atomes de carbones, et n est un entier compris entre 1 et 4, de préférence entre 2 et 4 et de manière très préférée n = 2 ou n = 4, les radicaux R pouvant être identiques ou différents. De manière préférée, ledit composé organométallique de l'étain est sélectionné dans le groupe constitué par le tétrabutyl-étain, le tétraméthyl-étain, le tétraéthyl-étain, le tétrapropyl-étain, le diphényl-étain, l'hydrure de tributyl-étain et le chlorure de tributyl-étain. De manière très préférée, ledit composé organométallique de l'étain est le tétrabutylétain.

Conformément à l'étape d) du procédé selon l'invention, on procède à au moins une étape d'activation dudit solide issu de ladite étape c) en présence d'au moins un gaz réducteur, de préférence l'hydrogène. Ladite étape d) d'activation est effectuée à une température comprise entre 100°C et 550°C, de manière préférée entre 150°C et 500°C, de manière encore plus préférée entre 330°C et 500°C pendant une durée comprise entre 1 heure et 40 heures, de manière préférée entre 3 heures et 20 heures. La montée jusqu'à cette température d'activation est généralement lente, par exemple elle est fixée entre 0,1°C/minute et 5°C/minute.

Le catalyseur obtenu à l'issue du procédé de préparation de l'invention est avantageusement utilisé directement à l'issue de ladite étape d) dans une unité réactionnelle réalisant la conversion d'une charge hydrocarbonée, en particulier dans une unité réactionnelle réalisant l'hydrogénation sélective d'une charge d'hydrocarbures polyinsaturés. Ledit catalyseur préparé selon le procédé de l'invention peut aussi être stocké à l'air puis réduit avant utilisation. La réduction consiste alors généralement en une montée lente de température, par exemple comprise entre 0,1°C/minute et 5°C/minute, sous courant de gaz réducteur, de préférence sous hydrogène, jusqu'à la température maximale de réduction, comprise entre 100°C et 600°C, de manière préférée entre 200°C et 500°C, suivie d'un maintien à cette température pendant une durée comprise entre 1 heure et 40 heures, de manière préférée entre 5 heures et 30 heures.

La présente invention a également pour objet un procédé d'hydrogénation sélective d'une charge d'hydrocarbures polyinsaturés, ledit procédé comprenant le passage de ladite charge dans au moins une unité réactionnelle pourvue d'au moins un catalyseur supporté comprenant au moins une phase métallique contenant de l'étain et du nickel préparé selon le procédé de préparation de l'invention.

Ladite charge d'hydrocarbures polyinsaturés, traitée dans le procédé d'hydrogénation sélective selon l'invention, est avantageusement une essence de vapocraquage comportant des hydrocarbures polyinsaturés contenant au moins 4 atomes de carbone et ayant un point d'ébullition final allant jusqu'à 220°C. Plus précisément, lesdits hydrocarbures polyinsaturés présents dans la charge traitée selon le procédé d'hydrogénation sélective de l'invention sont en particulier des composés dioléfiniques, des composés styréniques et des composés indéniques. Parmi les composés dioléfiniques, ladite charge contient notamment du butadiène, de l'isoprène et du cyclopentadiène. Parmi les composés styréniques, ladite charge contient notamment du styrène et de l'alpha-méthylstyrène. Parmi les composés indèniques, ladite charge contient notamment de l'indène.

Le procédé d'hydrogénation sélective selon l'invention vise à hydrogéner sélectivement lesdits hydrocarbures polyinsaturés présents dans ladite charge à traiter de manière à ce que les composés dioléfiniques soient partiellement hydrogénés en mono-oléfines et que les composés styréniques et indéniques soient partiellement hydrogénés en composés aromatiques correspondants.

L'effluent obtenu après la mise en oeuvre du procédé d'hydrogénation sélective de l'invention présente une teneur sensiblement amoindrie en hydrocarbures polyinsaturés, en particulier il présente une teneur amoindrie en composés dioléfiniques, en composés styréniques et en composés indéniques, tout en conservant une teneur en composés aromatiques (plus précisément une teneur en noyaux aromatiques) proche de celle présente dans par ladite charge d'hydrocarbures polyinsaturés. Ledit effluent est avantageusement valorisable comme base dans une essence ou utilisable comme base pour la valorisation de composés aromatiques.

Le procédé d'hydrogénation sélective selon l'invention est avantageusement effectué sous pression, en phase liquide, en présence d'une quantité d'hydrogène en faible excès par rapport à la valeur stoechiométrique permettant l'hydrogénation sélective des composés polyinsaturés présents dans la charge d'hydrocarbures, c'est-à-dire un excès compris généralement entre 5 et 30%. Le procédé d'hydrogénation sélective selon l'invention est mis en oeuvre à une température comprise entre 20°C et 200°C. La pression est généralement suffisante pour maintenir au moins 80 % poids de la charge à traiter en phase liquide à l'entrée de l'unité réactionnelle. Elle est généralement comprise entre 0,4 MPa et 5 MPa, plus avantageusement entre 1 MPa et 4 MPa. La vitesse spatiale horaire (définie comme le rapport du débit volumique de charge au volume de catalyseur), établie dans ces conditions est généralement comprise entre 0,2 h⁻¹ et 30 h⁻¹ et de préférence entre 1 h⁻¹ et 20 h⁻¹, et de manière encore plus préférée, entre 2 h⁻¹ et 10h⁻¹.

La mise en oeuvre technologique du procédé d'hydrogénation sélective est par exemple réalisée par injection, en courant ascendant ou descendant, de la charge et de l'hydrogène dans un réacteur à lit fixe. Elle peut également être avantageusement réalisée par l'implantation d'au moins dudit catalyseur supporté contenant de l'étain et du nickel dans une colonne de distillation réactive ou dans des réacteurs - échangeurs.

Les exemples qui suivent illustrent l'invention sans en limiter la portée.

### Exemple 1 (selon l'invention) : préparation d'un catalyseur B supporté sur alumine et contenant du nickel et de l'étain

Un catalyseur commercial de type Ni / alumine (LD 241, Axens, noté catalyseur A) est utilisé pour préparer un catalyseur à base de nickel et d'étain selon l'invention (catalyseur B). Ledit catalyseur commercial présente une teneur en nickel égale à 10% poids, une surface spécifique égale 70 m²/g, un volume poreux total égal à 0,63 cm³/g et un diamètre de billes compris entre 2 et 4 mm.

Le protocole de préparation du catalyseur B est le suivant. Une quantité de 0,5 g du catalyseur monométallique A est préalablement réduite sous courant d'hydrogène à 400°C durant 14 heures. Le dépôt de l'étain sur ledit catalyseur A réduit est réalisé en phase gazeuse : 9 mg de tétrabutylétain (Aldrich) sont injectés à température ambiante et sous atmosphère d'hydrogène sur le catalyseur A préalablement réduit. Après 3 heures de mise en contact, la température est portée à 450°C, sous courant d'hydrogène, à une vitesse de 2°C/minute. La température est alors maintenue à 450°C pendant 4 heures. Enfin, le catalyseur est refroidi et conservé à l'air.

Le catalyseur B ainsi obtenu contient 9,9 % poids de Ni et 0,6 % poids d'étain (d'après l'analyse élémentaire), ce qui correspond à un rapport molaire Sn / Ni de 0,030.

### Exemple 2 (comparatif) : préparation d'un catalyseur C supporté sur alumine et contenant du nickel et de l'étain, l'étain étant déposé en phase liquide.

Un catalyseur à base de nickel et d'étain (catalyseur C) est préparé à partir du catalyseur A selon le protocole suivant. Une quantité de 1 g du catalyseur monométallique A est préalablement réduite sous courant d'hydrogène à 400°C durant 14 heures. Le catalyseur C est préparé en mettant en contact 1 g du catalyseur monométallique A à l'état réduit avec 20 mL d'une solution de tétrabutylétain (Aldrich) dans l'heptane (Acros) (de concentration 4,5.10⁻⁴ mol/L), pendant 10 heures, à température ambiante, sous atmosphère d'hydrogène et sous agitation. Le solide est ensuite récupéré par filtration, lavé avec de l'heptane puis directement introduit dans l'autoclave en vue du test d'hydrogénation du styrène.

Le catalyseur C ainsi obtenu contient 9,9 % poids de Ni et 0,6 % poids d'étain (d'après l'analyse élémentaire), ce qui correspond à un rapport molaire Sn / Ni de 0,030.

### Exemple 3 (comparatif) : préparation d'un catalyseur D monométallique supporté sur silice contenant du nickel

Un catalyseur de type Ni / silice (catalyseur D) est préparé par échange cationique du sel [Ni(NH₃)₆]²⁺ sur la silice. La solution d'imprégnation est préparée en mélangeant une solution de nitrate de nickel (Aldrich) de concentration 7.10⁻³ mol/L à une solution ammoniacale (Aldrich) de concentration 0,4 mol/L. Une quantité de 4 g de silice (Aerosil-200, Degussa) est mise en contact avec 50 mL de la solution d'imprégnation durant 24 heures, à température ambiante et sous agitation. Le solide est ensuite récupéré par filtration, lavé avec de l'eau permutée, séché à l'étuve sous vide à 80°C puis 100°C et enfin traité sous courant d'hydrogène à 500°C avant d'être refroidi et conservé à l'air.

Le catalyseur monométallique D ainsi obtenu contient 11,7 % poids de Ni (d'après l'analyse élémentaire).

### Exemple 4 (selon l'invention) préparation d'un catalyseur E supporté sur silice et contenant du nickel et de l'étain

Un catalyseur à base de nickel et d'étain selon l'invention (catalyseur E) est préparé à partir du catalyseur D selon le protocole suivant. Une quantité de 0,41 g du catalyseur monométallique D est préalablement réduite sous courant d'hydrogène à 400°C durant 14 heures. Le dépôt de l'étain est réalisé en phase gazeuse : 10 mg de tétrabutylétain (Aldrich) sont injectés à température ambiante et sous atmosphère d'hydrogène sur le catalyseur D préalablement réduit. Après 3 heures de mise en contact, la température est portée à 350°C, sous courant d'hydrogène, à une vitesse de 2°C/minute. La température est alors maintenue à 350°C pendant 4 heures. Enfin, le catalyseur est refroidi et conservé à l'air.

Le catalyseur E ainsi obtenu contient 11,6 % poids de Ni et 0,8 % poids d'étain (d'après l'analyse élémentaire), ce qui correspond à un rapport molaire Sn / Ni de 0,034.

### Exemple 5 (selon l'invention) : préparation d'un catalyseur F supporté sur silice et contenant du nickel et de l'étain

Un autre catalyseur à base de nickel et d'étain selon l'invention (catalyseur F) est préparé à partir du catalyseur D selon le protocole suivant. Une quantité de 0,41 g du catalyseur monométallique D est préalablement réduite sous courant d'hydrogène à 400°C durant 14 heures. Le dépôt de l'étain est réalisé en phase gazeuse : 10 mg de tétrabutylétain (Aldrich) sont injectés à température ambiante et sous atmosphère d'hydrogène sur le catalyseur D préalablement réduit. Après 3 heures de mise en contact, la température est portée à 450°C, sous courant d'hydrogène, à une vitesse de 2°C/minute. La température est alors maintenue à 450°C pendant 4 heures. Enfin, le catalyseur est refroidi et conservé à l'air.

Le catalyseur F ainsi obtenu contient 11,6 % poids de Ni et 0,8 % poids d'étain (d'après l'analyse élémentaire), ce qui correspond à un rapport molaire Sn / Ni de 0,034.

### Exemple 6 (comparatif): préparation d'un catalyseur G supporté sur silice et contenant du nickel et de l'étain tel que le rapport molaire Sn / Ni = 0,25

Un autre catalyseur à base de nickel et d'étain (catalyseur G) est préparé à partir du catalyseur D selon le protocole suivant. Une quantité de 0,41 g du catalyseur monométallique D est préalablement réduite sous courant d'hydrogène à 400°C durant 14 heures. Le dépôt de l'étain est réalisé en phase gazeuse : 71 mg de tétrabutylétain (Aldrich) sont injectés à température ambiante et sous atmosphère d'hydrogène sur le catalyseur D préalablement réduit. Après 3 heures de mise en contact, la température est portée à 450°C, sous courant d'hydrogène, à une vitesse de 2°C/minute. La température est alors maintenue à 450°C pendant 4 heures. Enfin, le catalyseur est refroidi et conservé à l'air.

Le catalyseur G ainsi obtenu contient 11,5 % poids de Ni et 5,8 % poids d'étain (d'après l'analyse élémentaire), ce qui correspond à un rapport molaire Sn / Ni de 0,25.

### Exemple 7 (invention) : préparation d'un catalyseur H supporté sur silice et contenant du nickel et de l'étain tel que le rapport molaire Sn / Ni = 0,022

Un autre catalyseur à base de nickel et d'étain selon l'invention (catalyseur H) est préparé à partir du catalyseur D selon le protocole suivant. Une quantité de 0,41 g du catalyseur monométallique D est préalablement réduite sous courant d'hydrogène à 400°C durant 14 heures. Le dépôt de l'étain est réalisé en phase gazeuse : 6,5 mg de tétrabutylétain (Aldrich) sont injectés à température ambiante et sous atmosphère d'hydrogène sur le catalyseur D préalablement réduit. Après 3 heures de mise en contact, la température est portée à 450°C, sous courant d'hydrogène, à une vitesse de 2°C/minute. La température est alors maintenue à 450°C pendant 4 heures. Enfin, le catalyseur est refroidi et conservé à l'air.

Le catalyseur H ainsi obtenu contient 11,6 % poids de Ni et 0,5 % poids d'étain (d'après l'analyse élémentaire), ce qui correspond à un rapport molaire Sn / Ni de 0,022.

### Exemple 8 : Performances catalytique des catalyseurs A à H en hydrogénation du styrène.

Les propriétés catalytiques des catalyseurs préparés selon les exemples ci-dessus sont évaluées successivement dans un procédé d'hydrogénation du styrène. L'hydrogénation sélective du styrène conduit à l'éthylbenzène, cette hydrogénation constituant la réaction désirée. L'hydrogénation totale du styrène conduit à l'éthylcyclohexane, lequel est produit par une réaction successive indésirable.

L'hydrogénation du styrène est réalisée dans un autoclave de 100mL en acier inoxydable, muni d'une agitation mécanique à entraînement magnétique et pouvant fonctionner sous une pression maximale de 100 bar et des températures comprises entre 5°C et 200°C. Préalablement à son introduction dans l'autoclave, une quantité de 20 mg de catalyseur est réduite sous courant d'hydrogène à 400°C durant 14 heures, puis transvasée dans l'autoclave, à l'abri de l'air. Après ajout de 50 mL de n-heptane (Acros), l'autoclave est fermé, purgé, puis pressurisé sous 30 bar (0,3 MPa) d'hydrogène, et porté à la température initiale du test (40°C). Au temps t=0, 3,5 g de styrène (Aldrich) et un étalon interne sont introduits dans l'autoclave et l'agitation (500 tr/min) est mise en route. L'avancement de la réaction est suivi par prélèvement d'échantillons du milieu réactionnel à intervalles de temps réguliers. Ces échantillons sont analysés par chromatographie en phase gazeuse. Une fois que tout le styrène a été consommé, l'autoclave est porté rapidement à 130°C (en moins de 1 heure) : dans cette deuxième étape, la réaction qui a lieu est l'hydrogénation de l'éthylbenzène en éthylcyclohexane.

Les vitesses de réaction des deux réactions d'hydrogénation du styrène en éthylbenzène et d'hydrogénation de l'éthylbenzène en éthylcyclohexane sont définies comme les pentes à l'origine rapportées à la masse de catalyseur des courbes de l'évolution de la concentration en éthylbenzène au cours du temps. La vitesse de réaction r1 de l'hydrogénation du styrène en éthylbenzène est déterminée sur la première étape du test. La vitesse de réaction r2 de l'hydrogénation de l'éthylbenzène en éthylcyclohexane est déterminée sur la deuxième étape du test. La sélectivité du catalyseur envers le produit désiré, à savoir l'éthylbenzène, est évaluée par le rapport r1 / r2 ; le catalyseur est d'autant plus sélectif envers l'éthylbenzène que ce rapport est plus élevé.

Les vitesses de réaction d'hydrogénation du styrène et les sélectivités des catalyseurs préparés selon les exemples ci-dessus sont comparées dans les tableaux 1 et 2 à celles des catalyseurs monométalliques au Ni.

**Tableau 1 : Vitesses de réaction d'hydrogénation du styrène et sélectivités des catalyseurs supportés sur alumine. Comparaison par rapport au catalyseur monométallique A à base de nickel seulement.**

| Catalyseur | Rapport molaire Sn/Ni | Vitesse de réaction d'hydrogénation du styrène (r1)/(r1_{ref}) | Sélectivité (r1/ r2) / (r1_{ref} / r2_{ref}) |
|---|---|---|---|
| A | 0 | 1 | 1 |
| B | 0,030 | 1,8 | 3,2 |
| C | 0,030 | 0,8 | 1,5 |

**Tableau 2 : Vitesses de réaction d'hydrogénation du styrène et sélectivités des catalyseurs supportés sur silice. Comparaison par rapport au catalyseur monométallique D à base de nickel seulement.**

| Catalyseur | Rapport molaire Sn/Ni | Vitesse de réaction d'hydrogénation du styrène (r1) /(r1_{ref}) | Sélectivité (r1 /r2) / (r_{1ref}/ r2_{ref}) |
|---|---|---|---|
| D | 0 | 1 | 1 |
| E | 0,034 | 2,0 | 3,0 |
| F | 0,034 | 2,0 | 3,5 |
| G | 0,25 | 0,6 | 0,8 |
| H | 0,022 | 1,2 | 1,3 |

Dans les tableaux 1 et 2, r1_{ref}, respectivement r2_{ref}, correspondent aux vitesses de réaction r1 définie plus haut, respectivement r2 définie plus haut, mesurées avec le catalyseur monométallique correspondant.

Les catalyseurs B, E et F à base de nickel et d'étain, préparés selon le procédé de l'invention et ayant un rapport molaire Sn / Ni de l'ordre de 0,03, présentent des vitesses d'hydrogénation du styrène en éthylbenzène satisfaisantes, un peu plus élevées que celles des catalyseurs monométalliques au nickel correspondants (catalyseurs A et D), ce qui signifie que les performances sont maintenues et même supérieures vis-à-vis de l'hydrogénation de la double liaison présente dans le groupement en C2 porté par le noyau aromatique du styrène. Surtout les catalyseurs B, E et F présentent une sélectivité 3 à 4 fois plus élevée que celle des catalyseurs monométalliques au nickel correspondants (catalyseurs A et D). Aussi, pour les catalyseurs B, E et F, l'hydrogénation du cycle aromatique est ralentie par rapport à celle observée avec les catalyseurs A et D, lesquels favorisent la production d'éthylcyclohexane au détriment de l'éthylbenzène. Les catalyseurs B, E et F sont donc bien plus sélectifs envers l'éthylbenzène que les catalyseurs A et D. L'ajout d'étain en quantité contrôlée sur un catalyseur monométallique à base de nickel permet donc de ralentir la réaction d'hydrogénation du noyau aromatique du styrène tout en conservant de bonnes performances en hydrogénation de la double liaison.

Le catalyseur G à base de nickel et d'étain et ayant un rapport molaire Sn / Ni égal à 0,25 montre qu'une quantité trop importante d'étain est néfaste à la fois pour la vitesse d'hydrogénation du styrène en éthylbenzène et pour la sélectivité du catalyseur. Les catalyseurs B, E et F, préparés selon le procédé de l'invention dans des conditions telles que le rapport molaire Sn/Ni desdits catalyseurs est compris entre 0,01 et 0,2, sont bien plus sélectifs envers l'éthylbenzène que le catalyseur G, lequel favorise la production d'éthylcyclohexane au détriment de l'éthylbenzène.

Le catalyseur H à base de nickel et d'étain et ayant un rapport molaire Sn / Ni égal à 0,022 montre qu'une quantité faible d'étain permet une augmentation de la sélectivité du catalyseur même si cette augmentation est bien plus faible que celle obtenue avec les catalyseurs B, E ou F.

Le catalyseur C à base de nickel et d'étain, préparé en phase liquide en présence d'un solvant organique (heptane) et sans étape d'activation, présente également une sélectivité très faible.

Les catalyseurs B, E, F et H, préparés selon le procédé de l'invention, et présentant un rapport molaire compris entre 0,01 et 0,2, conduisent ainsi à des performances sensiblement améliorées en termes de sélectivité et de vitesse d'hydrogénation d'une double liaison lorsqu'ils sont mis en oeuvre dans un procédé d'hydrogénation sélective d'un composé alkénylaromatique tel que le styrène dans le cas présent. Lesdits catalyseurs B, E, F et H favorisent sensiblement l'hydrogénation de la double liaison présente dans le groupement en C2 porté par le cycle aromatique du styrène aux dépens de l'hydrogénation du noyau benzénique.

## Revendications

1. Procédé de préparation d'un catalyseur comprenant au moins un support poreux et au moins une phase métallique contenant du nickel et de l'étain dans une proportion telle que le rapport molaire Sn/Ni est compris entre 0,01 et 0,2, ledit procédé comprenant au moins successivement les étapes suivantes :
a) le dépôt de nickel sur au moins ledit support pour obtenir un catalyseur supporté monométallique à base de nickel,
b) la réduction dudit catalyseur monométallique en présence d'au moins un gaz réducteur,
c) le dépôt, en phase gazeuse et en présence d'au moins un gaz réducteur, d'au moins un composé organométallique de l'étain sur ledit catalyseur monométallique réduit et,
d) l'activation du solide issu de ladite étape c) en présence d'au moins un gaz réducteur pour obtenir un catalyseur supporté ayant une phase métallique à base de nickel et d'étain dans lequel l'étain représente de 0,02 à 15 % poids de la masse dudit catalyseur et le nickel de 1 à 50% poids de la masse dudit catalyseur.

2. Procédé de préparation selon la revendication 1 tel que ledit catalyseur présente un rapport molaire Sn/Ni compris entre 0,025 et 0,055.

3. Procédé de préparation selon la revendication 2 tel que ledit catalyseur présente un rapport molaire Sn/Ni compris entre 0,03 et 0,05.

4. Procédé de préparation selon l'une des revendications 1 à 3 tel que ledit support poreux présent dans ledit catalyseur est une alumine ou une silice.

5. Procédé de préparation selon l'une des revendications 1 à 4 tel que ladite étape b) est effectuée à une température comprise entre 100°C et 600°C, pendant une durée comprise entre 1 heure et 40 heures.

6. Procédé de préparation selon l'une des revendications 1 à 5 tel que ladite étape c) est réalisée en l'absence de solvant.

7. Procédé de préparation selon l'une des revendications 1 à 6 tel que ledit composé organométallique employé dans ladite étape c) répond de préférence à la formule Sn(R)ₙH₄₋ₙ où R est un radical alkyle ou un radical aryle ayant de 1 à 12 atomes de carbone, et n est un entier compris entre 1 et 4, les radicaux R pouvant être identiques ou différents.

8. Procédé de préparation selon la revendication 7 tel que ledit composé organométallique de l'étain est le tétrabutylétain.

9. Procédé de préparation selon l'une des revendications 1 à 8 tel que le gaz réducteur employé pour la mise en oeuvre de ladite étape c) est l'hydrogène.

10. Procédé de préparation selon l'une des revendications 1 à 9 tel que ladite étape d) est effectuée à une température comprise entre 100°C et 550°C pendant une durée comprise entre 1 heure et 40 heures.

11. Procédé d'hydrogénation sélective d'une charge d'hydrocarbures polyinsaturés comprenant le passage de ladite charge dans au moins une unité réactionnelle pourvue d'au moins un catalyseur supporté comprenant au moins une phase métallique préparé selon le procédé de préparation selon l'une des revendications 1 à 10.

12. Procédé d'hydrogénation sélective selon la revendication 11 tel que ladite charge d'hydrocarbures polyinsaturés est une essence de vapocraquage comportant des hydrocarbures polyinsaturés contenant au moins 4 atomes de carbone et ayant un point d'ébullition final allant jusqu'à 220°C.

13. Procédé d'hydrogénation sélective selon la revendication 12 tel que lesdits hydrocarbures polyinsaturés sont des composés dioléfiniques, des composés styréniques et des composés indéniques.

14. Procédé d'hydrogénation sélective selon l'une des revendications 11 à 13 tel qu'il est mis en oeuvre à une température comprise entre 20°C et 200°C, sous une pression comprise entre 0,4 MPa et 5 MPa, avec une vitesse spatiale horaire (définie comme le rapport du débit volumique de charge au volume de catalyseur), comprise entre 0,2 h⁻¹ et 30 h⁻¹.

## Claims

1. A process for preparing a catalyst comprising at least one porous support and at least one metallic phase containing nickel and tin in a proportion such that the Sn/Ni molar ratio is in the range 0.01 to 0.2, said process comprising at least the following steps in succession:
a) depositing nickel on at least said support in order to obtain a supported nickel-based monometallic catalyst;
b) reducing said monometallic catalyst in the presence of at least one reducing gas;
c) depositing, in gas phase and in the presence of at least one reducing gas, at least one organometallic tin compound onto said reduced monometallic catalyst; and
d) activating the solid derived from said step c) in the presence of at least one reducing gas in order to obtain a supported catalyst having a metallic phase based on nickel and tin in which tin represents 0.02% to 15% by weight of the mass of said catalyst and nickel represents 1% to 50% by weight of the mass of said catalyst.

2. A preparation process according to claim 1, in which said catalyst has a Sn/Ni molar ratio in the range 0.025 to 0.055.

3. A preparation process according to claim 2, in which said catalyst has a Sn/Ni molar ratio in the range 0.03 to 0.05.

4. A preparation process according to one of claims 1 to 3, in which said porous support present in said catalyst is an alumina or a silica.

5. A preparation process according to one of claims 1 to 4, in which said step b) is carried out at a temperature in the range 100°C to 600°C, for a period in the range 1 hour to 40 hours.

6. A preparation process according to one of claims 1 to 5, in which said step c) is carried out in the absence of solvent.

7. A preparation process according to one of claims 1 to 6, in which said organometallic compound employed in said step c) preferably has formula Sn(R)ₙH₄₋ₙ where R is an alkyl radical or an aryl radical containing 1 to 12 carbon atoms and n is a whole number in the range 1 to 4, radicals R possibly being identical or different.

8. A preparation process according to claim 7, in which said organometallic tin compound is tetrabutyltin.

9. A preparation process according to one of claims 1 to 8, in which the reducing gas employed to carry out said step c) is hydrogen.

10. A preparation process according to one of claims 1 to 9, in which said step d) is carried out at a temperature in the range 100°C to 550°C for a period in the range 1 hour to 40 hours.

11. A process for the selective hydrogenation of a feed of polyunsaturated hydrocarbons, comprising passing said feed into at least one reaction unit provided with at least one supported catalyst comprising at least one metallic phase prepared in accordance with the preparation process according to one of claims 1 to 10.

12. A selective hydrogenation process according to claim 11, in which said feed of polyunsaturated hydrocarbons is a steam cracked gasoline comprising polyunsaturated hydrocarbons containing at least 4 carbon atoms and having an end point of up to 220°C.

13. A selective hydrogenation process according to claim 12, in which said polyunsaturated hydrocarbons are diolefin compounds, styrene compounds and indene compounds.

14. A selective hydrogenation process according to one of claims 11 to 13, carried out at a temperature in the range 20°C to 200°C, at a pressure in the range 0.4 MPa to 5 MPa, at an hourly space velocity (defined as the ratio of the volume flow rate of feed to the volume of catalyst) in the range 0.2 h⁻¹ to 30 h⁻¹.

## Patentansprüche

1. Verfahren zur Herstellung eines Katalysators umfassend mindestens einen porösen Träger und mindestens eine metallische Phase, welche Nickel und Zinn in einem derartigen Anteil enthält, dass das Molverhältnis Sn/Ni im Bereich von 0,01 bis 0,2 liegt, wobei das Verfahren mindestens nacheinander die folgenden Schritte umfasst:
a) Abscheiden von Nickel mindestens auf dem Träger, um einen monometallischen geträgerten Katalysator auf Nickelbasis zu erhalten,
b) Reduktion des monometallischen Katalysators in Gegenwart mindestens eines reduzierenden Gases,
c) Abscheiden, in der Gasphase und in Gegenwart mindestens eines reduzierenden Gases, mindestens einer metallorganischen Zinnverbindung auf dem reduzierten monometallischen Katalysator, und
d) Aktivierung des Feststoffs, der in Schritt c) erhalten wurde, in Gegenwart mindestens eines reduzierenden Gases, um einen geträgerten Katalysator mit einer metallischen Phase auf Nickel- und Zinnbasis zu erhalten, bei welchem das Zinn 0,02 bis 15 Gewichts-% der Masse des Katalysators und das Nickel 1 bis 50 Gewichts-% der Masse des Katalysators ausmachen.

2. Herstellungsverfahren nach Anspruch 1, derart, dass der Katalysator ein Sn/Ni-Molverhältnis im Bereich von 0,025 bis 0,055 aufweist.

3. Herstellungsverfahren nach Anspruch 2, derart, dass der Katalysator ein Sn/Ni-Molverhältnis im Bereich von 0,03 bis 0,05 aufweist.

4. Herstellungsverfahren nach einem der Ansprüche 1 bis 3, derart, dass es sich bei dem porösen Träger, der in dem Katalysator vorliegt, um ein Aluminiumoxid oder ein Siliciumdioxid handelt.

5. Herstellungsverfahren nach einem der Ansprüche 1 bis 4, derart, dass der Schritt b) bei einer Temperatur im Bereich von 100 °C bis 600 °C über eine Zeitdauer im Bereich von 1 Stunde bis 40 Stunden durchgeführt wird.

6. Herstellungsverfahren nach einem der Ansprüche 1 bis 5, derart, dass der Schritt c) ohne Lösemittel durchgeführt wird.

7. Herstellungsverfahren nach einem der Ansprüche 1 bis 6, derart, dass die metallorganische Verbindung, welche in Schritt c) verwendet wird, vorzugsweise der Formel Sn(R)ₙH₄₋ₙ entspricht, wobei R ein Alkylrest oder ein Arylrest mit 1 bis 12 Kohlenstoffatomen ist und n eine ganze Zahl im Bereich von 1 bis 4 ist, wobei die Reste R gleichartig oder verschiedenartig sein können.

8. Herstellungsverfahren nach Anspruch 7, derart, dass es sich bei der metallorganischen Zinnverbindung um Tetrabutylzinn handelt.

9. Herstellungsverfahren nach einem der Ansprüche 1 bis 8, derart, dass es sich bei dem reduzierenden Gas, welches zur Durchführung des Schrittes c) verwendet wird, um Wasserstoff handelt.

10. Herstellungsverfahren nach einem der Ansprüche 1 bis 9, derart, dass der Schritt d) bei einer Temperatur im Bereich von 100 °C bis 550 °C über eine Zeitdauer im Bereich von 1 Stunde bis 40 Stunden durchgeführt wird.

11. Verfahren zur selektiven Hydrierung einer Charge mehrfach ungesättigter Kohlenwasserstoffe, wobei es das Einleiten der Charge in mindestens eine Reaktionseinheit umfasst, welche mit mindestens einem geträgerten Katalysator versehen ist, der mindestens eine metallische Phase umfasst, die gemäß dem Herstellungsverfahren nach einem der Ansprüche 1 bis 10 hergestellt ist.

12. Verfahren zur selektiven Hydrierung nach Anspruch 11, derart, dass es sich bei der Charge mehrfach ungesättigter Kohlenwasserstoffe um ein Benzin aus dem Dampfcrackverfahren handelt, welches mehrfach ungesättigte Kohlenwasserstoffe aufweist, die mindestens 4 Kohlenstoffatome enthalten und einen Endsiedepunkt von bis zu 220 °C haben.

13. Verfahren zur selektiven Hydrierung nach Anspruch 12, derart, dass es sich bei den mehrfach ungesättigten Kohlenwasserstoffen um diolefinische Verbindungen, styrolartige Verbindungen und um indenartige Verbindungen handelt.

14. Verfahren zur selektiven Hydrierung nach einem der Ansprüche 11 bis 13, derart, dass es bei einer Temperatur im Bereich von 20 °C bis 200 °C, unter einem Druck im Bereich von 0,4 MPa bis 5 MPa, mit einer stundenbezogenen Raumgeschwindigkeit (definiert als Verhältnis zwischen dem Volumendurchsatz der Charge und dem Katalysatorvolumen) im Bereich von 0,2 h⁻¹ und 30 h⁻¹ durchgeführt wird.
